# EUROPÄISCHE PATENTSCHRIFT

(11) **EP 0 983 302 B1**
(45) Veröffentlichungstag und Bekanntmachung des Hinweises auf die Patenterteilung: **26.04.2006**
(21) Anmeldenummer: 98934774.5
(22) Anmeldetag: 22.05.1998
(51) Int. Cl.: C07K 16/28, A61K 39/395, G01N 33/577, G01N 33/574

(54) **Mutiertes ScFv-Fragment des OKT-3 Antikörpers**
Mutated ScFv-Fragment of the OKT-3 antibody
Fragment ScFv muté de l'anticorps OKT-3

(30) Priorität: 23.05.1997 DE 19721700
(43) Veröffentlichungstag der Anmeldung: 08.03.2000
(73) Patentinhaber: Deutsches Krebsforschungszentrum Stiftung des öffentlichen Rechts, 69120 Heidelberg (DE)
(72) Erfinder: LITTLE, Melvyn, D-69151 Neckargemünd (DE); KIPRIYANOV, Sergey, D-69121 Heidelberg (DE); MOLDENHAUER, Gerhard, D-69120 Heidelberg (DE)
(74) Vertreter: Schüssler, Andrea
(86) Internationale Anmeldenummer: PCT/DE1998/001409
(87) Internationale Veröffentlichungsnummer: WO 1998/052975

(56) Entgegenhaltungen:
- WO-A-94/28027
- WO-A-94/29350
- S. KIPRIYANOV ET AL.: "Two amino acid mutations in an anti-human CD3 single-chain Fv antibody fragment that affect the yield on bacterial secretion but not the affinity." PROTEIN ENGINEERING, Bd. 10, Nr. 4, April 1997, Seiten 445-453, XP002079905 Oxford, GB
- S. KIPRIYANOV ET AL.: "Rapid detection of recombinant antibody fragments directed against cell-surface antigens by flow cytometry." JOURNAL OF IMMUNOLOGICAL METHODS, Bd. 196, Nr. 1, 13. September 1996, Seiten 51-62, XP002079906 Amsterdam, NL in der Anmeldung erwähnt
- S. DÜPEL ET AL.: "Isolation of IgG antibody Fv-DNA from various mouse and rat hybridoma cell lines using the polymerase chain reaction with a simple set of primers." JOURNAL OF IMMUNOLOGICAL METHODS, Bd. 175, Nr. 1, 30. September 1994, Seiten 89-95, XP002079907 Amsterdam, NL in der Anmeldung erwähnt
- D. KROON ET AL.: "Identification of sites of degradation in a therapeutic monoclonal antibody by peptide mapping." PHARMACEUTICAL RESEARCH, Bd. 9, Nr. 11, November 1992, Seiten 1386-1393, XP002079908 Stuttgart, Deutschland

## Beschreibung

Die vorliegende Erfindung betrifft ein durch eine Punktmutation an Position H100A mutiertes OKT3-Antikörperfragment, Verfahren zu seiner Herstellung sowie seine Verwendung.

OKT3 ist ein aus Maus stammender monoklonaler Antikörper vom IgG 2a-Typ, der ein Epitop einer ε-Untereinheit des menschlichen CD3-Komplexes erkennt (Kung et al., Science 206, S. 347-349 (1979); Van Wauwe et al., J. Immunol. 124, S. 2708-2713 (1980); Transy et al., Eur. J. Immunol. 19, S. 947-950 (1989)). Das Verfahren, den monoklonalen Antikörper aus dem entsprechenden Hybridom zu erhalten, ist in diesen Druckschriften im Detail beschrieben. Außerdem wurde die OKT3 produzierende Hybridomazellinie am 26. April 1979 unter der ATCC-Nummer CRL 8001 bei der American Type Culture Collection, 12301 Parklawn Drive, Rockville, MD, 20852 von der Inhaberin des EP-Patents 0 018 795 hinterlegt. OKT3 wird seit langem benutzt, um eine T-Zellantwort zu unterdrücken und dadurch die Abstoßung von Transplantaten zu verhindern (Thistlethwaite et al., Transplantation 38, S. 695-701 (1984); Woodle et al., Transplantation 51, S. 1207-1212 (1991)). Andererseits kann durch OKT3 auch eine T-Zell-Aktivierung und Proliferation ausgelöst werden, die Effektorzellen anregt, was bei der adoptiven Krebs-Immuntherapie eingesetzt werden kann (Yannelly et al., J. Immunol. Meth. 1, S. 91-100 (1990)). OKT3 wurde sowohl alleine als auch als Komponente eines bispezifischen Antikörpers eingesetzt, um cytotoxische T-Lymphozyten gegen Tumorzellen oder Virus-infizierte Zellen zu richten (Nitta et al., Lancet 335, S. 368-376 (1990); Sanna et al., Bio/Technology 13, S. 1221-1224 (1995)). Außerdem sind auch humanisierte Versionen des OKT3-monoklonalen Antikörpers, die in COS-Zellen exprimiert wurden, bekannt (Woodle et al., J. Immunol. 148, S. 2756-2763 (1992); Adair et al., Human. Antibod. Hybridomas, S. 41-47 (1994)). Bisher bestand aber das Problem, daß OKT3 keine ausreichende Stabilität aufweist und inbesondere sich nicht in bekannten rekombinanten Expressionssystemen stabil und in genügender Menge exprimieren läßt.

Die Aufgabe der vorliegenden Erfindung bestand deshalb darin, OKT3 rekombinant zu exprimieren und ein Antikörperfragment zu erhalten, das eine befriedigende Stabilität aufweist.

Die Aufgabe wird durch die Gegenstände der Patentansprüche gelöst.

Von den Erfindern wurde gefunden, daß durch Einbringen einer Punktmutation an Position H100A der Aminosäuresequenz von OKT3 die Stabilität um ein Vielfaches zunimmt. Diese Punktmutation betrifft den Austauch von Cystein gegen eine andere polare Aminosäure, bevorzugt Serin, in der Aminosäuresequenz von OKT3.

Zur Herstellung eines erfindungsgemäßen Antikörperfragments wird von mRNA aus frisch subklonierten Hybridomazellen von OKT3 ausgegangen. Die cDNA wird nach den dem Fachmann bekannten Methoden, die beispielsweise in Dübel et al., J. Immunol. Methods 175, S. 89-95 (1994) beschrieben wurden, hergestellt. Die für die variable Domäne der leichten Kette kodierende DNA kann mittels PCR unter Verwendung geeigneter Primer, z.B. mittels der Primer Bi5 and Bi8, die an den aminoterminalen Teil der konstanten Domäne der κ-Kette und die "Framework 1 (FR1)-Region der variablen Domäne der κ-Kette hybridisieren, hergestellt werden (Dübel et al., vgl. vorstehend). Für die Amplifikation der DNA, die für die variable Domäne der schweren Kette codiert, können beispielsweise der Primer Bi4, der an den aminoterminalen Teil der konstanten Domäne 1 der γ-Kette hybridisiert (Dübel et al., vgl. vorstehend) und der Primer Bi3f, der an die FR1-Region der schweren Kette hybridisiert (Gotter et al., Tumor Targeting 1, S. 107-114 (1995), verwendet werden.

Die amplifizierte DNA wird danach in einen für die Sequenzierung und für "site-specific mutagenesis" geeigneten Vektor, wie er dem Fachmann bestens bekannt sind, inseriert. Beispielsweise kann der von der Firma Stratagene vertriebene Vektor pCR-Skript SK(+) verwendet werden. Mutationen werden in der von OKT3 kannt sind, inseriert. Beispielsweise kann der von der Firma Stratagene vertriebene Vektor pCR-Skript SK(+) verwendet werden. Mutationen werden in der von OKT3 stammenden v_{H}-Domäne durch gerichtete Mutagenese ("site specific mutagenesis") eingebracht. Die dafür notwendigen Bedingungen sind dem Fachmann bekannt, beispielsweise auch in Kunkel et al., Meth. Enzymol. 154, S. 367-382 (1987) beschrieben. Die Aminosäuresubstitution an der Position H100A von OKT3 (Austausch von Cystein) wird geeigneterweise unter Verwendung des Primers SK1 5'-GTAGTCAAGGCTGTAATGATCATC durchgeführt, falls ein Austausch gegen Serin an dieser Position ausgeführt werden soll.

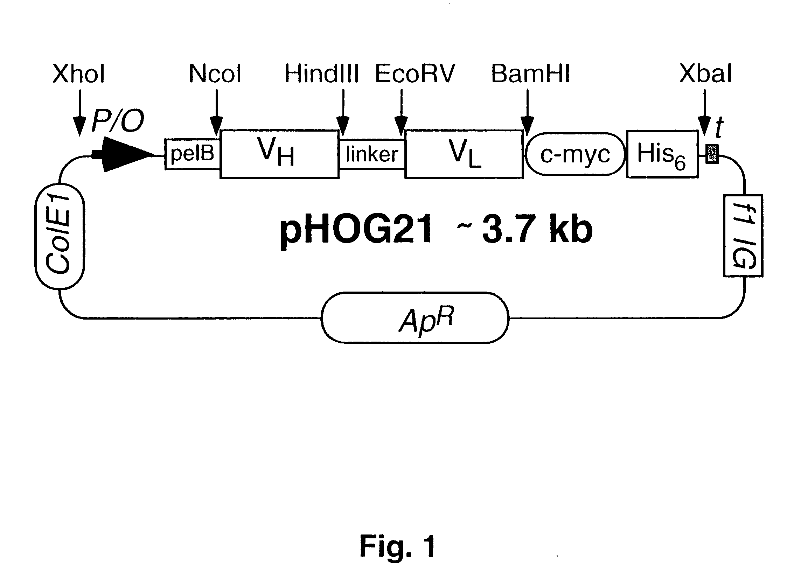

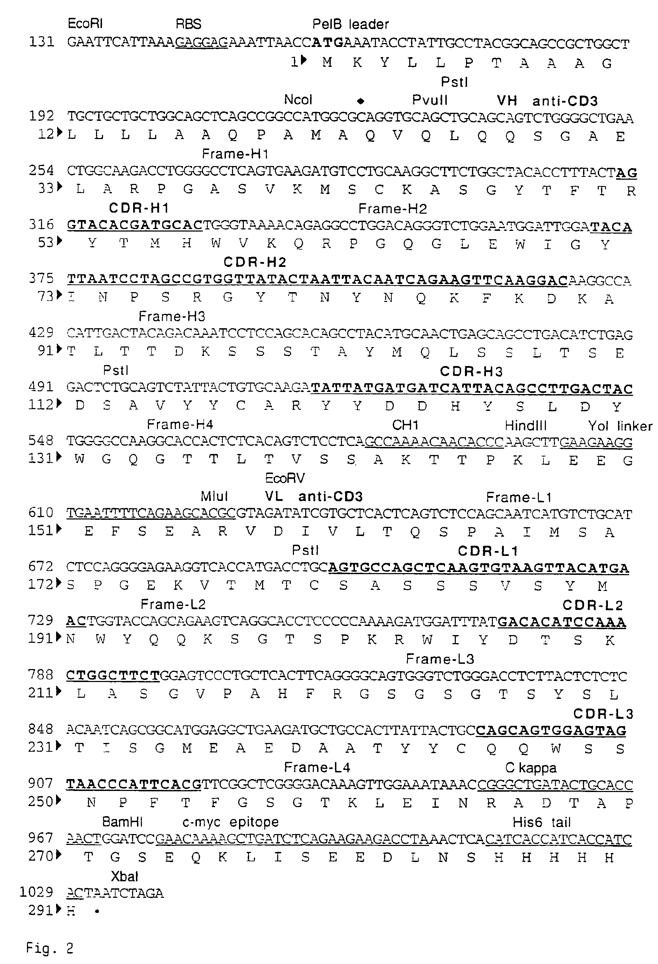

Die so veränderte DNA kann danach in einen Vektor bzw. Expressionsvektor kloniert werden. Beispiele solcher sind dem Fachmann bekannt. Im Falle eines Expressionsvektors sind dies pGEMEX, pUC-Derivate oder pET3b. Für die Expression in Hefe sind z.B. pY100 und Ycpad 1 zu nennen, während für die Expression in tierischen Zellen z.B. pKCR, pEFBOS, cDM8 und pCEV4 anzugegeben sind. Für die Expression in Insektenzellen eignet sich besonders der Baculovirus-Expressionsvektor pAcSGHisNT-1. Die Expression in E. coli ist erfindungsgemäß bevorzugt, wofür vorzugsweise der in Fig.1 gezeigte Vektor pHOG21 (Kipriyanov et al., J. Immunol. Methods 196, S. 51-62 (1996) eingesetzt wird, worin das mutierte OKT3-Einzelketten(scFv)-Gen als NcoI/BamHI DNA-Fragment insertiert ist. Es kommt zur Expression eines an der Position 100 A (Kabat-Nummerierungssystem) mutierten einzelkettigen Antikörpers OKT3, der die in Fig. 2 gezeigte Sequenz aufweist.

Der Fachmann kennt geeignete Zellen, um eine in einem Expressionsvektor vorliegende DNA zu exprimieren. Beispiele solcher Zellen umfassen die E. coli-Stämme HB101, DH1, x1776, JM101, JM109, B121 und SG 13009, den Hefestamm Saccharomyces cerevisiae und die tierischen Zellen 3T3, FM3A, CHO, COS, Vero und Hela sowie die Insektenzellen sf9. Bevorzugt ist die Verwendung der von der Firma Stratagene vertriebenen XL1-Blue E. coli-Zellen.

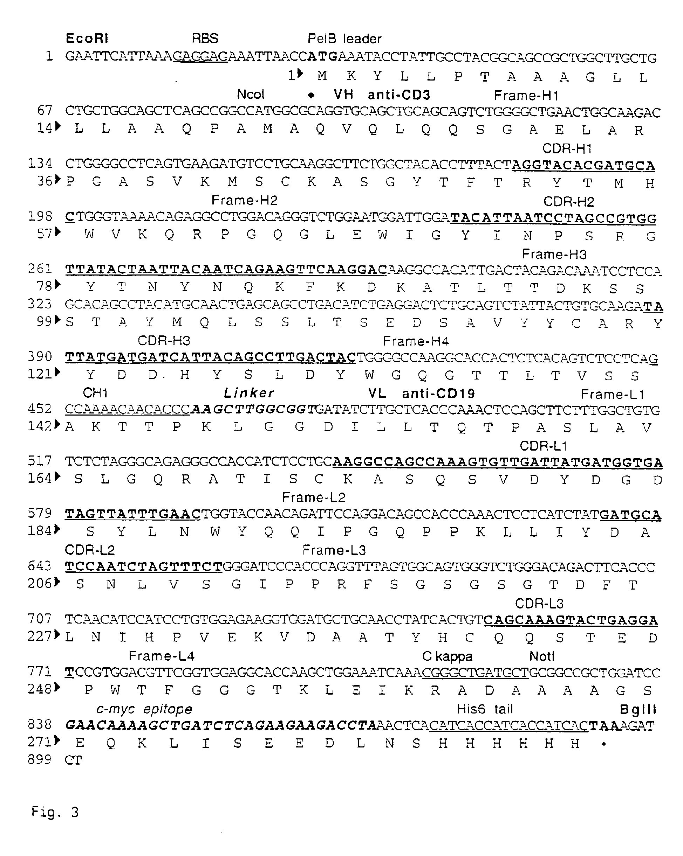

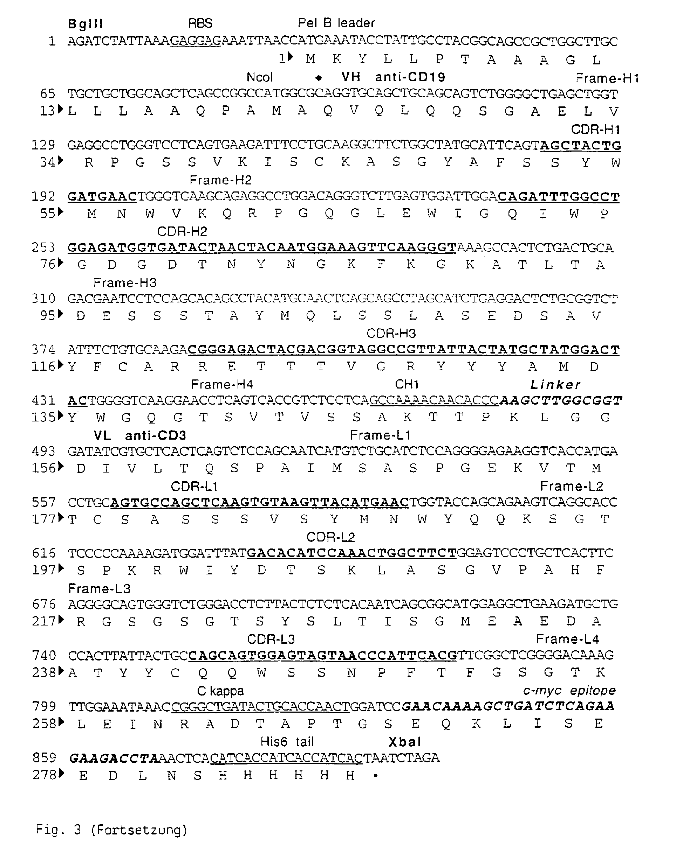

Der Fachmann weiß, in welcher Weise eine DNA in einen Expressionsvektor daß die DNA in Form eines Fusionsproteins exprimiert werden kann, beispielsweise in Form eines His-Fusionsproteins. Die dafür notwendige Information ist im vorzugsweise verwendeten Plasmid pHOG21 enthalten. Weiter kann die mutierte Form von OKT3 in Form eines bispezifischen Antikörpers, z.B. in Verbindung mit einem Antikörper gegen menschlichen CD19-Komplex vorliegen. Die Sequenz eines solchen bispezischen Antikörpers ist in Fig. 3 gezeigt.

Erfindungsgemäße Antikörperfragmente zeichnen sich dadurch aus, daß sie mittels rekombinanter Methoden in ausreichender Menge hergestellt werden können und eine im Vergleich zum unmutierten monoklonalen Antikörper OKT3 größere Stabilität aufweist. Diese äußert sich beispielsweise darin, daß das mutierte Antikörperfragment auch noch nach einem Monat Lagerung bei 4°C in PBS kaum von seiner ursprünglichen Bindungsaffinität eingebüßt hat, wohingegen OKT3 unter diesen Bedingungen bereits deutlich an Bindungsaffinität verloren hat (46%). Außerdem hat das erfindungsgemäße Antikörperfragment den Vorteil, daß es als Einzelkettenantikörper (scFv) eine schnellere Blut-Clearance und eine bessere Tumorpenetration aufweist. Weiter sind ScFv's sehr nützliche Moleküle, um Arzneistoffe, Toxine oder Radionuklide an Tumorstellen zu bringen, was in der Tumordiagnostik und - therapie wichtig ist.

Die vorliegende Erfindung wird weiter anhand der Figuren beschrieben.
- Fig. 1:: Plasmid pHOG21
wobei die verwendeten Abkürzungen folgende Bedeutungen haben:
Ap^{R}: Ampicillin-Resistenzgen
c-myc: Sequenz codierend für ein Epitop, das durch den monoklonalen Antikörper 9E10 (Cambridge Research Biochemicals, Cambridge, Großbritannien) erkannt wird
ColE1: Ursprung der DNA-Replikation
fl IG: Intergene Region des f1-Phagen
His₆: Sequenz codierend für 6 Histidinreste
linker: Sequenz codierend für 17 Aminosäuren, die die v_{H}- und v_{L}-Domäne verbindet
pelB: Signalpeptidsequenz für bakterielle Pektatlyase
P/O: Wildtyp-Lac-Promotor/Operator
- Fig. 2:: Nukleotid- und abgeleitete Aminosäuresequenz des mutierten OKT3-Einzelkettenantikörpers
- Fig. 3:: Bispezifischer Antikörper zusammengesetzt aus mutiertem OKT3 und anti-CD 19

Die Erfindung wird weiter anhand des Beispiels erläutert.

### BEISPIEL 1: Herstellung eines erfindungsgemäßen Antikörperfragments

Die Isoloation von mRNA aus frisch subklonierten Hybridomazellen von OKT3 und die cDNA-Synthese wurde, wie in "Dübel et al., J. Immunol. Methods 175, S. 89-95 (1994)" beschrieben durchgeführt. Die für die variable Domäne der leichten Kette kodierende DNA wurde mittels PCR unter Verwendung der Primer Bi5 and Bi8, die an den aminoterminalen Teil der konstanten Domäne der κ-Kette und die "Framework 1 (FR1)-Region der variablen Domäne der κ-Kette hybridisieren, hergestellt (Dübel et al., vgl. vorstehend). Für die Amplifikation der DNA, die für die variable Domäne der schweren Kette codiert, wurde der Primer Bi4, der an den aminoterminalen Teil der konstanten Domäne 1 der γ-Kette hybridisiert (Dübel et al., vgl. vorstehend) und der Primer Bi3f, der an die FR1-Region der schweren Kette hybridisiert (Gotter et al., Tumor Targeting 1, S. 107-114 (1995), verwendet. Das 50 *µ*l Reaktionsgemisch enthielt 10 pmol jedes Primers und 50 ng Hybridoma cDNA, 100 *µ*M jedes der dNTPs, 1x Vent-Puffer (Boehringer Mannheim), 5 *µ*g BSA und 1 U Vent DNA-Polymerase. Es wurden 30 Zyklen je 1 Minute bei 95°C, 1 Min. bei 55°C und 2 Minuten bei 75°C in einem PCR-Thermozykler durchgeführt. Die amplifizierte DNA wurde mit einem OIA-quick PCR-Reiningungskit (Qiagen, Hilden) gereinigt. je 1 Minute bei 95°C, 1 Min. bei 55°C und 2 Minuten bei 75°C in einem PCR-Thermozykler durchgeführt. Die amplifizierte DNA wurde mit einem QIA-quick PCR-Reiningungskit (Qiagen, Hilden) gereinigt.

Die amplifizierte DNA wurde danach in den von der Firma Stratagene vertriebenen Vektor pCR-Skript SK(+), der mit dem Restriktionsenzym Srfl geschnitten worden war, "blunt-end" ligiert. Mutationen wurden in der von OKT3 stammenden v_{H}-Domäne durch gerichtete Mutagenese ("site specific mutagenesis") eingebracht (Kunkel et al., Meth. Enzymol. 154, S. 367-382 (1987)). Die Aminosäuresubstitution an der Position H100A von OKT3 (Austausch von Cystein gegen Serin) wurde unter Verwendung des Primers SK1 5'-GTAGTCAAGGCTGTAATGATCATC durchgeführt.

Für die Expression der erhaltenen mutierten DNA wurde der in Fig.1 gezeigte Vektor pHOG21 (Kipriyanov et al., J. Immunol. Methods 196, S. 51-62 (1996) eingesetzt, worin das mutierte OKT3-Einzelketten(scFv)-Gen als NcoI/BamHI DNA-Fragment inseriert ist. XL1-Blue E. coli-Zellen (Stratagene) wurden mit diesem Expressionsvektor transformiert und über Nacht in 2xYT-Medium mit 50 *µ*g/ml Ampicillin und 100 mM Glucose (2xYT_{GA}) bei 37°C wachsengelassen. Verdünnungen (1:50) der Übernachtkulturen in 2xYT_{GA} wurden bei 37 °C unter Schütteln bei 37°C wachsengelassen. Sobald die Kulturen OD₆₀₀ = 0,8 erreichten, wurden die Bakterien durch Zentrifugation bei 1500 g für 10 Minuten and 20°C pelletiert und im gleichen Volumen frischem 2xYT-Medium enthaltend 50µg/ml Ampicillin und 0,4 M Sucrose resuspendiert. Es wurde IPTG auf eine Endkonzentration von 0,1 mM zugesetzt und das Wachstum bei Raumtemperatur für 20 Std. fortgesetzt. Die Zellen wurden durch Zentrifugation bei 5000g für 10 Minuten und 4°C geerntet. Der Kulturüberstand wurde auf Eis aufbewahrt. Um lösliche periplasmatische Proteine zu isolieren, wurden die pelletierten Bakterien in eiskaltem 50 mM Tris-HCl, 20% Sucrose, 1 mM EDTA, pH 8,0 (5% des Ursprungsvolumens) aufgenommen. Nach einer Stunde Inkubation auf Eis mit gelegentlichem Rühren wurden Spheroplasten bei 30000 g für 30 Minuten und 4°C abzentrifugiert, wobei der lösliche periplasmatische Extrakt als Überstand und die Spheroplasten plus unlösliches periplasmatisches Material als Pellet anfielen. Der vorstehend auf Eis aufbewahrte Kulturüberstand und der lösliche periplasmatische Extrakt wurden kombiniert und durch eine zusätzliche Zentrifugation (30000 g, 4°C, 40 Min.) geklärt. Nach Filtrationen durch Glasfilter mit einer Porengröße von 10-16 *µ*m und dann 0,2 *µ*m wurde das Volumen 10-fach durch Konzentration mit Amicon YM10 Membranen (Amicon, Witten). Der konzentrierte Überstand wurde durch Zentrifugation geklärt und gegen 50 mM Tris-HCl, 1 M NaCl, pH 7,0 bei 4°C dialysiert. Immobilisierte Metall-Affinitätschromatografie (IMAC) wurde bei 4°C unter Verwendung einer 5 ml Säule von chelatisierender Sepharose (Pharmacia) beladen mit Ni²⁺ und equilibriert mit 50 mM Tris-HCl, 1 M NaCl, pH 7,0 (Startpuffer) durchgeführt. Auf der Säule adsorbiertes Material wurde mit 50 mM Tris-HCl, 1 M NaCl, 250 mM Imidazol, pH 7,0 eluiert. Nach Pufferwechsel zu 50 mM MES, pH 6,0 wurde das Protein weiter auf einer Mono S lonenaustauschsäule (Pharmacia) gereinigt. Der erfindungsgemäße gereinigte scFv-Antikörper wurde in PBS (15 mM Natriumphosphat, 0,15 M NaCl, pH 7,4) dialysiert. Für einen längere Aufbewahrung wurde der Antikörper in Anwesenheit von BSA (Endkonzentration 10 mg/ml) eingefroren und bei -80°C gelagert.

## Patentansprüche

1. ScFv-Fragment eines Antikörpers umfassend eine Bindungsdomäne, die durch einen Austausch von Cystein gegen eine andere polare Aminosäure an Position H100A gemäß dem Kabat-Nummerierungssystem des unter der Bezeichnung OKT3 bekannten Antikörpers, der in Form von Hydridomazellen unter der Nummer ATCC CRL 8001 hinterlegt ist, **gekennzeichnet** ist.

2. Fragment nach Anspruch 1, **dadurch gekennzeichnet, daß** die polare Aminosäure Serin ist.

3. Fragment nach einem der Ansprüche 1 oder 2, **dadurch gekennzeichnet, daß** dieser die in Fig. 2 angegebene Sequenz aufweist.

4. Verfahren zur Herstellung des Fragments nach einem der Ansprüche 1 bis 3 **gekennzeichnet durch** die folgenden Schritte:
a) Gewinnen von mRNA aus frisch subklonierten Hybridomazellen von OKT3 und Umschreibung zu cDNA
b) Amplifikation der für die variablen Domänen der leichten und schweren Kette kodierenden DNA mittels PCR unter Verwendung geeigneter Primer,
c) Klonierung der unter b) erhaltenen DNA in einen zur gerichteten Mutagenese geeigneten Vektor sowie Einführung der gewünschten Mutation unter Verwendung geeigneter Primer,
d) Insertion der unter c) erhaltenen mutierten DNA in einem Expressionsvektor und Expression in einem geeigneten Expressionssystem.

5. Verfahren nach Anspruch 4, wobei der in Schritt c) verwendete Vektor pCR-Skript SK(+) ist.

6. Verfahren nach Anspruch 4 oder 5, wobei in Schritt c) der Primer 5'-GTAGTCAAGGCTGTAATGATCATC verwendet wird.

7. Verfahren nach einem der Ansprüche 4 bis 6, wobei die Expression in XL1-Blue E.coli-Zellen erfolgt.

8. Verwendung des Fragments nach einem der Ansprüche 1 bis 3 zur Herstellung eines Medikaments zur Verminderung oder Ausschaltung einer Transplantatabstoßung durch einen Organtransplantatempfänger.

## Claims

1. A scFv-fragment of an antibody comprising a binding domain, **characterized by** an exchange of cysteine for another polar amino acid at position H100A according to the Kabat numbering system of the OKT3 antibody known under this name and deposited in the form of hybridoma cells under the number ATCC CRL 8001.

2. The fragment according to claim 1, **characterized in that** the polar amino acid is serine.

3. The fragment according to claim 1 or 2, **characterized in that** it includes the sequence indicated in figure 2.

4. A method for the production of the fragment according to any one of claims 1 to 3, **characterized by** the steps of:
a) obtaining mRNA from freshly subcloned hybridoma cells of OKT3 and transcription into cDNA,
b) amplification of the DNA coding for the variable domains of the light and heavy chains by means of PCR using suitable primers,
c) cloning of the DNA obtained in b) into a vector adapted for site-specific mutagenesis as well as introduction of the desired mutation using suitable primers,
d) insertion of the mutated DNA obtained in c) in an expression vector and expression in a suitable expression system.

5. The method according to claim 4, wherein the vector used in step c) is pCR-Skript SK(+).

6. The method according to claim 4 or 5, wherein the primer 5'-GTAGTCAAGGCTGTAATGATCATC is used in step c).

7. The method according to any one of claims 4 to 6, wherein the expression takes place in XL1-Blue *E. coli* cells.

8. Use of the fragment according to any one of claims 1 to 3 for the manufacturing of a medicament for reducing or eliminating a transplant rejection by an organ transplant recipient.

## Revendications

1. Fragment ScFv d'un anticorps englobant un domaine de liaison qui est **caractérisé par** un échange de cystéine contre un autre acide aminé polaire en position H100A selon le système de numérotation Kabat de l'anticorps connu sous la désignation OKT3 et déposé sous la forme de cellules hybrides sous le numéro ATCC CRL 8001.

2. Fragment selon la revendication 1, **caractérisé en ce que** l'acide aminé polaire est représenté par la sérine.

3. Fragment selon la revendication 1 ou 2, **caractérisé en ce qu'**il possède la séquence indiquée à la figure 2.

4. Procédé de préparation d'un fragment selon l'une quelconque des revendications 1 à 3, **caractérisé en ce qu'**il comprend les étapes suivantes :
a) produire du mARN à partir de cellules hybrides fraîchement subclonées de OTK3 et transformation en cADN,
b) amplification de l'ADN codant des domaines variables des chaînes faibles et fortes au moyen de PCR en utilisant le primaire approprié,
c) clonage de l'ADN obtenu en b) en un vecteur approprié par mutagénèse dirigée ainsi qu'une introduction de la mutation souhaitée en utilisant le primaire approprié,
d) insertion de l'ADN muté obtenu à l'étape c) dans un vecteur d'expression et expression dans un système d'expression approprié.

5. Procédé selon la revendication 4, dans lequel le vecteur utilisé dans l'étape c) est pCR-Skript SH(+).

6. Procédé selon la revendication 4 ou 5, dans lequel le primaire utilisé dans l'étape c) est 5' - GTAGTCAAGGCTGTAATGATCATC.

7. Procédé selon l'une quelconque des revendications 4 à 6, dans lequel l'expression est réalisée dans des cellules XL1-Blue E.Coli.

8. Utilisation du fragment selon l'une quelconque des revendications 1 à 3 dans la préparation d'un médicament pour diminuer ou éliminer un rejet de transplant par un receveur d'un transplant d'organe.
